# EUROPÄISCHE PATENTSCHRIFT

(11) **EP 2 100 145 B1**
(45) Veröffentlichungstag und Bekanntmachung des Hinweises auf die Patenterteilung: **28.02.2018**
(21) Anmeldenummer: 07856070.3
(22) Anmeldetag: 10.12.2007
(51) Int. Cl.: G01N 33/68

(54) **DIAGNOSE UND RISIKOSTRATIFIZIERUNG VON HERZINSUFFIZIENZ MITTELS NEUROPHYSIN**
DIAGNOSIS AND RISK STRATIFICATION OF CARDIAC INSUFFICIENCY USING NEUROPHYSIN
DIAGNOSTICS ET STRATIFICATION DES RISQUES DE L'INSUFFISANCE CARDIAQUE PAR LA NEUROPHYSIN

(30) Priorität: 08.12.2006 DE 102006058266
(43) Veröffentlichungstag der Anmeldung: 16.09.2009
(73) Patentinhaber: B.R.A.H.M.S GmbH, 16761 Hennigsdorf (DE)
(72) Erfinder: BERGMANN, Andreas, 12351 Berlin (DE); STRUCK, Joachim, 13465 Berlin (DE)
(74) Vertreter: Simandi, Claus
(86) Internationale Anmeldenummer: PCT/DE2007/002215
(87) Internationale Veröffentlichungsnummer: WO 2008/067806

(56) Entgegenhaltungen:
- EP-A- 1 628 136
- WO-A-2004/006860
- WO-A-2008/049422
- DE LORGERIL M ET AL: "[Possible role of vasopressin in ischemic accidents related to tobacco consumption]" ARCHIVES DES MALADIES DU COEUR ET DES VAISSEAUX JAN 1984, Bd. 77, Nr. 1, Januar 1984 (1984-01), Seiten 71-75, XP009101839 ISSN: 0003-9683
- NORTH W G ET AL: "ISOLATION AND PARTIAL CHARACTERIZATION OF 2 HUMAN NEUROPHYSINS THEIR USE IN THE DEVELOPMENT OF SPECIFIC RADIO IMMUNOASSAYS" JOURNAL OF CLINICAL ENDOCRINOLOGY AND METABOLISM, Bd. 51, Nr. 4, 1980, Seiten 884-891, XP009101737 ISSN: 0021-972X in der Anmeldung erwähnt

## Beschreibung

Die Erfindung betrifft ein Verfahren zur Diagnose und / oder Risikostratifizierung von Herzinsuffizienz, wobei eine Bestimmung des Markers Neurophysin oder Fragment oder Teilpeptid davon an einem zu untersuchenden Patienten durchgeführt wird. Die Erfindung betrifft vorteilhafte Markerkombinationen enthaltend Neurophysin. Weiterhin betrifft die Erfindung eine diagnostische Vorrichtung und Kit zur Durchführung des Verfahrens.

In Europa kommen jährlich etwa eine Million Patienten mit dem Symptom der akuten Atemnot in die Notaufnahmen von Kliniken. Die Atemnot ist ein Leitsymptom vieler Erkrankungen und lässt sich in ca. 35-47% der Fälle auf eine Herzinsuffizienz zurückführen (Januzzi JL Jr, Camargo CA, Anwaruddin S, Baggish AL, Chen AA, Krauser DG, Tung R, Cameron R, Nagurney JT, Chae CU, Lloyd-Jones DM, Brown DF, Foran-Melanson S, Sluss PM, Lee-Lewandrowski E, Lewandrowski KB, The N-terminal Pro-BNP investigation of dyspnea in the emergency department (PRIDE) study, Am J Cardiol. 95(8) (2005), pp. 948-954 und Maisel AS, Krishnaswamy P, Nowak RM, McCord J, Hollander JE, Duc P, Omland T, Storrow AB, Abraham WT, Wu AH, Clopton P, Steg PG, Westheim A, Knudsen CW, Perez A, Kazanegra R, Herrmann HC, McCullough PA; Breathing Not Properly Multinational Study Investigators, Rapid measurement of B-type natriuretic peptide in the emergency diagnosis of heart failure, N Engl J Med. 347(3) (2002), pp. 161-167).

Im Anfangsstadium bemerkt der Patient oft nur wenig von der Herzinsuffizienz. Unbehandelt nimmt die Erkrankung in der Regel an Schwere zu und führt im Spätstadium zu völliger körperlicher Erschöpfung bereits in Ruhe. Die Unterversorgung aller Körperorgane, einschließlich des Herzmuskels selbst, kann in diesem Stadium zum Tode führen. Ist die Erkrankung erst einmal fortgeschritten, ist die Lebenserwartung auch unter optimaler Therapie stark vermindert (ca. 30% Todesfälle pro Jahr). Es ist daher wichtig, eine Herzschwäche möglichst früh zu erkennen und ihre Ursachen konsequent anzugehen.

Um mit einer geeigneten Therapie zu beginnen, bedarf es daher einer frühen Diagnose und Differenzierung der zugrunde liegenden Erkrankung bereits im Frühstadium und in der Not- und Intensivmedizin. Aufgrund unspezifischer Symptome (Luftnot, Husten) ist sowohl die Differenzierung und Abgrenzung der Herzinsuffizienz von anderen Erkrankungen häufig erschwert.

Mittels Bestimmung der Plasmakonzentration des brain natriuretic peptide (BNP bzw. NTproBNP) steht ein Test zur Verfügung, der auch in der Alltagsroutine für die Diagnostik einer Herzinsuffizienz eingesetzt wird (Maisel et al. (supra)).

Im Stand der Technik ist Neurophysin als Marker beschrieben für die Nikotinaufnahme (Robinson AG. Isolation, assay, and secretion of individual human neurophysins. J Clin Invest 1975;55:360-7), Krebs- und nicht-Krebs-assoziierter SIADH (Syndrome of inappropriate ADH secretion) und nephrogenem Diabetes insipidus (Pullan PT, Clappison BH, Johnston CI. Plasma vasopressin and human neurophysins in physiological and pathological states associated with changes in vasopressin secretion. J Clin Endocrinol Metab 1979; 49: 580-7; North WG, LaRochelle FT, Jr., Melton J, Mills RC. Isolation and partial characterization of two human neurophysins: their use in the development of specific radioimmunoassays. J Clin Endocrinol Metab 1980; 51:884-91).

Es besteht im Stand der Technik ein hohes Bedürfnis weitere Marker für die Indikation Herzinsuffizienz zu identifizieren und bereitzustellen und der klinischen Praxis zuzuführen.

In EP 1 628 136 A1 wird der Marker Copeptin und dessen Eignung in der medizinischen Diagnostik offenbart, wobei ein Verbund eines Vasopressin-NeurophysinII-Copeptin-Precursor beschrieben wird, der jedoch von Neurophysin II verschieden ist.

De Lorgeril M. et al: ("[Possible role of vasopressin in ischemic accidents related to tobacco consumption]", ARCHIVES DES MALADIES DU COEUR ET DES VAISSEAUX JAN 1984, Bd. 77, Nr. 1, Januar 1984 (1984-01), Seiten 71-75) beschreiben ein "Nikotin-stimulierendes Neurophysin" zur Diagnose von "Sudden-Death".

WO 2004/006860 A1 offenbart einen monoklonalen Antikörper zum Nachweis von Pro-Vasopressin samt Diagnose von Krebs.

Daher ist es Aufgabe der vorliegenden Erfindung ein Verfahren zur Diagnose und / oder Risikostratifizierung von Herzinsuffizienz bereitzustellen.

Nachteilig an bekannten Diagnoseverfahren unter Verwendung der bisher bekannten Markern ist jedoch, dass eine frühzeitige und vollständige Erfassung von Risikopatienten nicht gelingt und daher eine Risikostratifizierung nur ungenügend erfolgt. Eine der Erfindung weitere zugrunde liegende Aufgabe besteht daher darin, ein Verfahren zur Risikostratifizierung von Herzinsuffizienz zu entwickeln, das eine verbesserte Erfassung von Risikopatienten ermöglicht.

Die Aufgabe wird durch ein Verfahren zur Diagnose und / oder Risikostratifizierung von Herzinsuffizienz gelöst, wobei eine Bestimmung des Markers Neurophysin II oder ein Fragment oder Teilpeptid davon an einem zu untersuchenden Patienten durchgeführt wird (nachstehend erfindungsgemäßes Verfahren). Der Schutzbereich der Erfindung wird durch die Ansprüche definiert. Rahmen dieser Erfindung wird unter "Herzinsuffizienz" ein akutes oder chronisches Unvermögen des Herzens, die Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, deren ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Chrisian Mewis, Reimer Riessen und Ioakim Spyridopoulos, 2. unveränderte Auflage, Thieme 2006). Weiterhin erfindungsgemäß umfasst sind hypertensive Herzkrankheit mit (kongestiver) Herzinsuffizienz, Hypertensive Herz- und Nierenkrankheit mit (kongestiver) Herzinsuffizienz, Primäre Rechtsherzinsuffizienz, Sekundäre Rechtsherzinsuffizienz, Linksherzinsuffizienz ohne Beschwerden CNYHA-Stadium I), Linksherzinsuffizienz mit Beschwerden bei stärkerer Belastung (NYHA-Stadium II), Linksherzinsuffizienz mit Beschwerden bei leichterer Belastung (NYHA-Stadium III), Linksherzinsuffizienz mit Beschwerden in Ruhe (NYHA-Stadium IV) .

Daher umfasst das beschriebene Verfahren ebenfalls die oben genannten Indikationen. Alle genannten Indikationen werden zudem z.B. im Pschyrembel, De Gruyter, Berlin 2004 beschrieben.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Herzinsuffizienz mit dem Ziel eines möglichst günstigen Verlaufs zu ermöglichen. Eine erfindungsgemäße Risikostratifizierung erlaubt in Folge ein effektives Behandlungsverfahren, die betreffend einer Herzinsuffizienz gegeben sind.
Besonders vorteilhaft kann insbesondere in Fällen der Notfall- und /oder Intensivmedizin mittels des erfindungsgemäßen Verfahren eine sichere Diagnose erfolgen. Das erfindungsgemäße Verfahren ermöglicht klinische Entscheidungen, die zu einem schnellen Therapieerfolg führen. Solche klinische Entscheidungen umfassen ebenfalls weiterführende Behandlung mittels Arzneimitteln zur Behandlung oder Therapie von Herzinsuffizienz, solche wie ACE-Hemmer, AT1-Antagonisten: Blocker des Angiotensin-II-Rezeptors (Subtyp 1), Betablocker Bisoprolol, Carvedilol, Metoprolol und Nebivolol, Vasopressin-Rezeptor-Antagonisten, Aldosteronantagonisten ab NYHA-Stadium III, Kalzium-Sensitizer (Levosimendan).

In einer weiteren bevorzugten Ausführungsform betrifft das erfindungsgemäße Verfahren daher die Therapiesteuerung einer Herzinsuffizienz.

Daher betrifft die Erfindung ebenfalls ein Verfahren zur Risikostratifizierung von Patienten, insbesondere zur Stratifizierung von Patienten für klinische Entscheidungen, vorzugsweise in der zeitlich kritischen Intensivmedizin oder Notfallmedizin und zur Hospitalisierung von Patienten.

In einer weiteren bevorzugten Ausführungsform des erfindungsgemäßen Verfahrens erfolgt die Diagnose zur Prognose, zur differentialdiagnostischen Früherkennung und Erkennung, zur Beurteilung des Schweregrades und zur therapiebegleitenden Verlaufsbeurteilung einer Herzinsuffizienz.

In einer weiteren Ausführungsform des erfindungsgemäßen Verfahrens wird dem zu untersuchenden Patienten Körperflüssigkeit, insbesondere Blut entnommen, wahlweise Vollblut oder Serum, und die Diagnose erfolgt *in vitro*/*ex vivo,* d.h. außerhalb des menschlichen oder tierischen Körpers. Aufgrund der Bestimmung des Markers Neurophysin und seiner vorhandenen Menge in mindestens einer Patientenprobe kann die Diagnose erfolgen.

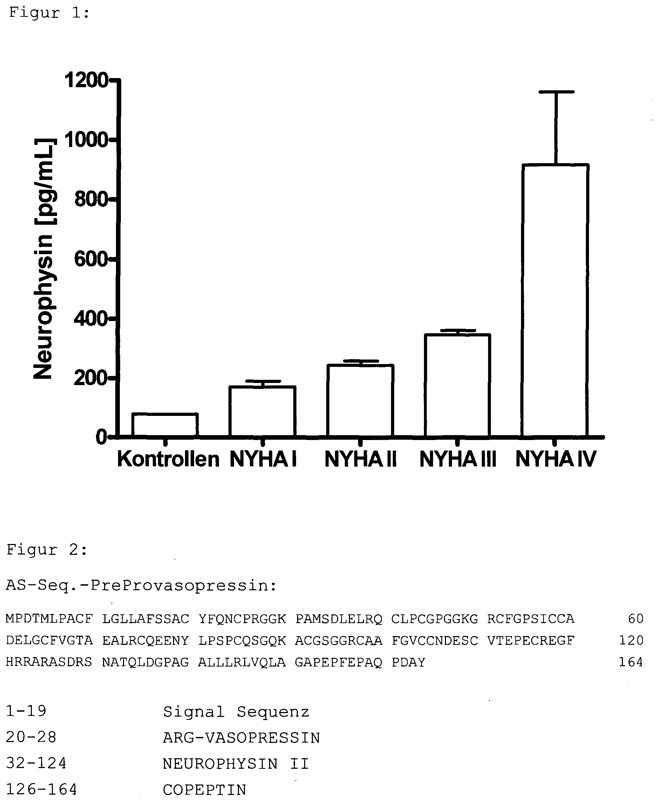

Im Rahmen dieser Erfindung wird unter "Neurophysin II" ((Fragment: AS 32-124 des Preprovasopressins; siehe Figur 2) ein freies 93 Aminosäuren enthaltendes Polypeptid/Protein verstanden (93AS: SEQ ID No. 1: AMSDLELRQC LPCGPGGKGR CFGPSICCAD ELGCFVGTAE ALRCQEENYL PSPCQSGQKA CGSGGRCAAF GVCCNDESCV TEPECREGFH RRA) Auch Fragmente oder Teilpeptide davon sind bekannt. Ferner kann dieses Polypeptid posttranslationale Modifikationen aufweisen, wie Glykolisierung, Lip(o)idisierung oder Derivatisierungen. Neurophysin ist überraschend stabil im Plasma.

In einer weiteren Ausführungsform kann die Bestimmung von Neurophysin II zusätzlich mit weiteren Markern erfolgen und zwar vorzugsweise solche, die bereits auf eine Herzinsuffizienz hinweisen und einen synergetischen Effekt von Markerkombinationen enthaltend Neurophysin II im erfindungsgemäßen Verfahrens erlauben.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker aus mindestens einem Marker aus der Gruppe Kreatinkinase, Myeloperoxidase, Copeptin, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin (proEnd), pro-Leptin, pro-Neuropeptid-Y, pro-Somatostatin, pro- Neuropeptid-YY, pro-Opiomelanocortin, pro-Adrenomedullin (proADM), Copeptin oder jeweils eine Teilsequenz davon.
Der ischämische Marker kann aus mindestens einem Marker aus der Gruppe Troponin I und T, CK-MB ausgewählt sein. Darüber hinaus kann der neurohormonaler Marker mindestens ein natriuretisches Protein sein, insbesondere ANP (bzw. ANF), proANP, NT-proANP, BNP, proBNP, NT-proBNP oder jeweils eine Teilsequenz davon.

Besonders bevorzugt sind Markerkombinationen von Neurophysin II mit einem Prohormon, insbesondere Copeptin, und / oder BNP, proBNP, NT-proBNP.

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung.

Ferner betrifft die Erfindung die Verwendung von Neurophysin II oder Fragment oder Teilpeptid davon und ggfs. weitere Marker, wie oben ausgeführt, zur Diagnose und / oder Risikostratifizierung von Herzinsuffizienz.

Eine weitere Aufgabe ist die Bereitstellung einer entsprechenden diagnostischen Vorrichtung oder dessen Verwendung zur Durchführung des erfindungsgemäßen Verfahrens.

Im Rahmen dieser Erfindung wird unter einer diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Die Erfindung betrifft zudem ein Kit zur Diagnose oder zur Risikostratifizierung von Herzinsuffizienz,
enthaltend Nachweisreagenzien zur Bestimmung des Markers Neurophysin II oder Fragment oder Teilpeptid davon und ggfs. oben genannten weiteren Marker. Solche Nachweisreagenzien umfassen z.B. Antikörper, Immunfluoreszenz etc.

Nachfolgende Beispiele und Figuren dienen zur näheren Erläuterung der Erfindung, jedoch ohne die Erfindung auf diese Beispiele und Figuren zu beschränken.

### Beispiele und Figuren:

Patienten, die sich mit dem Leitsymptom der Atemnot in der Notaufnahme eines Krankenhauses vorgestellt haben, wurde während der Eingangsuntersuchung eine Blutprobe entnommen. Durch Zentrifugation gewonnenes EDTA-Plasma wurde aliquotiert und bis zur Vermessung von Neurophysin bei -80°C gelagert. Gemäß früheren Beschreibungen (Pullan (supra)) wurde ein Radioimmunoassay für Neurophysin erstellt: Neurohypophysäres Neurophysin wurde isoliert und quantifiziert. Damit wurden Kaninchen immunisiert und so hochtitrige anti-Neurophysin Antiseren gewonnen. Für den Immunoassay wurde das hochtitrigste Antiserum in einer Konzentration von 1:100.000 eingesetzt. Gereinigtes Neurophysin wurde mit der Chloramin T-Methode radiojodiert und als Tracer im Assay eingesetzt. Als Standards dienten Verdünnungen von gereinigtem Neurophysin in normalem Pferdeserum. Der Assay wurde wie folgt durchgeführt: 50 µl Probe bzw. Standard wurden mit 100 µl Tracer (12.000 dpm pro Bestimmung) und 100 µl verdünntem anti-Neurophysin Antiserum gemischt und 24 Stunden bei 4°C inkubiert. Als Puffer wurde verwendet: 100 mM Natrium Phosphat, pH 7.5, 0.1 % BSA. An Antikörper gebundener Tracer wurde von freiem Tracer getrennt, indem 60% Ethanol zugesetzt wurden und dann 15 Minuten bei 4°C und 5.000 g zentrifugiert wurde. Der Überstand wurde verworfen, und die im Pellet verbliebene Radioaktivität wurde bestimmt. Die Auswertung erfolgte mithilfe der Software Multicalc. Der Assay hatte eine analytische Nachweisgrenze von 22 pg/ml und einen Messbereich bis 400 pg/ml. Mit dem Assay wurden Plasmaproben verschiedener Patienten, wie weiter unten erläutert, gemessen. Proben mit Messwerten >400 pg/ml wurden in geeigneten Verdünnungen gemessen, so dass Messwerte innerhalb des Messbereichs erzielt wurden.

Die Diagnose der Herzinsuffizienz bei den untersuchten Patienten basierte auf dem Framingham Score (McKee PA, Castelli WP, McNamara P and Kannel WB, The natural history of congestive heart failure: the Framingham study, N Engl J Med 285 (1971), pp. 1441-1446) für Herzinsuffizienz plus einer echographischen Evidenz für eine systolische oder diastolische Dysfunktuktion.

Des Weiteren wurde gesunden Individuen ohne bekannte Erkrankung Blut abgenommen und durch Zentrifugation EDTA-Plasma gewonnen.

### Klinische Wertigkeit

### Normalbereich

Neurophysin Konzentrationen wurden in Proben von gesunden Kontrollpersonen (n=200) bestimmt. Der Median lag bei 70,3 pg/ml, der kleinste gemessene Wert bei 8,8, der größte bei 220 pg/ml, die 95% Percentilen bei 36,5 bzw. 135 pg/ml.

### Herzinsuffizienz / Schweregrad

Es wurden Neurophysin Konzentrationen bei Patienten mit chronischer oder akut dekompensierter Herzinsuffizienz gemessen. Neurophysin Konzentrationen waren mit dem Schweregrad der Herzinsuffizienz assoziiert: Die Mittelwerte der Neurophysin Konzentrationen bei den vier Schweregrad-Kategorien NYHA I-IV waren: 171,4, 243,4, 346,9 bzw. 918,1 pg/ml (siehe Figur 1).

### Chronische Herzinsuffizienz / Diagnose

Von einem Kollektiv von 258 Patienten mit chronischer Herzinsuffizienz sowie 200 gesunden Kontrollen wurden Neurophysin Werte bestimmt. Die Receiver-Operator-Characteristics Analyse ergab eine AUC von 0,89. Bei einem Cut-off-Wert von 213 pg/ml ergab sich eine Sensitivität von 48% bei einer Spezifität von 98%. Bei einem Cut-off-Wert von 136,1 pg/ml ergab sich eine Sensitivität von 68,2% bei einer Spezifität von 95%.

### Chronische Herzinsuffizienz / Prognose

Von einem Kollektiv von 258 Patienten mit chronischer Herzinsuffizienz wurden Neurophysin Werte bestimmt. Die Patienten wurden über einen mittleren Zeitraum von 360 Tagen beobachtet. In diesem Zeitraum starben 80 Patienten, 178 überlebten. Durch Receiver-Operator-Characteristics Analyse wurde der beste Cut-off-Wert (definiert als das größte Produkt aus Sensitivität und Spezifität) zur Prognose der Mortalität ermittelt: 247 pg/ml. Bei diesem Cut-off-Wert betrug die Sensitivität der Prognose 59,5%, die Spezifität betrug 65,2%. Die Likelihood Ratio betrug bei einem Cut-off-Wert von 247 pg/ml: 1.7 (siehe nachfolgende Tabelle).

| | <247 pg/ml | >247 pg/ml |
|---|---|---|
| Überlebende | 116 | 62 |
| Tote | 32 | 47 |

### Akute Herzinsuffizienz / Diagnose

Von einem Kollektiv von 125 Patienten mit akuter Atemnot wurden Neurophysin Werte bestimmt. Von den 125 Patienten hatten 69 Patienten Herzinsuffizienz. Die Receiver-Operator-Characteristics Analyse für die Differentialdiagnose der Herzinsuffizienz ergab eine AUC von 0,61. Bei einem Cut-off-Wert von 4940 pg/ml ergab sich eine Sensitivität von 6,6% bei einer Spezifität von 98%. Bei einem Cut-off-Wert von 3000 pg/ml ergab sich eine Sensitivität von 11,7% bei einer Spezifität von 95%.

### Akute Herzinsuffizienz / Prognose

Von einem Kollektiv von 69 Patienten mit akut dekompensierter Herzinsuffizienz wurden Neurophysin Werte bestimmt. Die Patienten wurden über einen Zeitraum von 360 Tagen beobachtet. In diesem Zeitraum starben 21 Patienten, 48 überlebten. Durch Receiver-Operator-Characteristics Analyse wurde der beste Cut-off-Wert (definiert als das größte Produkt aus Sensitivität und Spezifität) zur Prognose der Mortalität ermittelt: 885 pg/ml. Bei diesem Cut-off-Wert betrug die Sensitivität der Prognose 57,1%, die Spezifität betrug 75%. Die Likelihood Ratio betrug bei einem Cut-off-Wert von 885 pg/ml: 2,3 (siehe nachfolgende Tabelle).

| | <885 pg/ml | >885 pg/ml |
|---|---|---|
| Überlebende | 36 | 12 |

**Tabelle: Klassifikation der New York Heart Association (NYHA)**

| | | |
|---|---|---|
| Tote | 9 | 12 |

- NYHA I: Keine körperliche Einschränkung. Alltägliche körperliche Belastung verursacht keine inadäquate Erschöpfung, Rhythmusstörungen, Luftnot oder Angina Pectoris.
- NYHA II: Leichte Einschränkung der körperlichen Belastbarkeit. Keine Beschwerden in Ruhe. Erschöpfung, Rhythmusstörungen, Luftnot oder Angina pectoris bei alltäglicher körperlicher Belastung.
- NYHA III: Höhergradige Einschränkung der körperlichen Leistungsfähigkeit bei gewohnter Tätigkeit. Keine Beschwerden in Ruhe. Erschöpfung, Rhythmusstörungen, Luftnot oder Angina pectoris bei geringer körperlicher Belastung.
- NYHA IV: Beschwerden bei allen körperlichen Aktivitäten und in Ruhe; Bettlägrigkeit.

## Patentansprüche

1. Verfahren zur in-vitro Diagnose und / oder Risikostratifizierung von Herzinsuffizienz, **dadurch gekennzeichnet,**
**dass** eine Bestimmung des Markers Neurophysin II (SEQ ID No. 1) von einem zu untersuchenden Patienten durchgeführt wird.

2. Verfahren nach Anspruch 1, **dadurch gekennzeichnet, dass** die Herzinsuffizienz eine chronische Herzinsuffizienz, hypertensive Herzkrankheit mit (kongestiver) Herzinsuffizienz, hypertensive Herz- und Nierenkrankheit mit (kongestiver) Herzinsuffizienz, Primäre Rechtsherzinsuffizienz, Sekundäre Rechtsherzinsuffizienz, Linksherzinsuffizienz ohne Beschwerden (NYHA-Stadium I), Linksherzinsuffizienz mit Beschwerden bei stärkerer Belastung (NYHA-Stadium II), Linksherzinsuffizienz mit Beschwerden bei leichterer Belastung (NYHA-Stadium III), Linksherzinsuffizienz mit Beschwerden in Ruhe (NYHA-Stadium IV) umfasst.

3. Verfahren zur in-vitro Diagnose und / oder Risikostratifizierung von Herzinsuffizienz nach einem der Ansprüche 1 bis 2 zur Durchführung von klinischen Entscheidungen, insbesondere weiterführende Behandlung und Therapie mittels Arzneimitteln, insbesondere in der Intensivmedizin oder Notfallmedizin und zur Hospitalisierung des Patienten.

4. Verfahren zur in-vitro Diagnose von Herzinsuffizienz nach einem der Ansprüche 1 bis 3 zur Prognose, zur differentialdiagnostischen Früherkennung und Erkennung, zur Beurteilung des Schweregrades und zur therapiebegleitenden Verlaufsbeurteilung einer Herzinsuffizienz

5. Verfahren nach einem der vorherigen Ansprüche,
**dadurch gekennzeichnet,**
**dass** zusätzlich eine Bestimmung mindestens eines weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

6. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

7. Verfahren nach einem der vorherigen Ansprüche, **dadurch gekennzeichnet, dass** der cardiovaskuläre Marker aus mindestens einem Marker aus der Gruppe Kreatinkinase, Myeloperoxidase, Copeptin, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-Y, pro-Somatostatin, pro-Neuropeptid-YY, pro-Opiomelanocortin, pro-Adrenomedullin (proADM), Copeptin oder jeweils eine Teilsequenz davon ausgewählt ist.

8. Verfahren nach einem der vorherigen Ansprüche, **dadurch gekennzeichnet, dass** der ischämischer Marker aus mindestens einem Marker aus der Gruppe Troponin I und T, CK-MB ausgewählt ist.

9. Verfahren nach einem der vorherigen Ansprüche, **dadurch gekennzeichnet, dass** der neurohormonaler Marker mindestens ein natriuretisches Protein ist, insbesondere ANP (bzw. ANF), proANP, NT-proANP, BNP, proBNP, NT-proBNP oder eine Teilsequenz davon ist.

10. Verfahren nach einem der vorherigen Ansprüche,
**dadurch gekennzeichnet,**
**dass** parallele oder simultane Bestimmungen der Marker durchgeführt werden.

11. Verfahren nach einem der vorherigen Ansprüche,
**dadurch gekennzeichnet,**
**dass** die Bestimmungen an mindestens einer Patientenprobe durchgeführt werden.

12. Verfahren nach einem der vorherigen Ansprüche,
**dadurch gekennzeichnet,**
**dass** die Bestimmungen an einem Analyseautomaten, insbesondere mittels einem Kryptor, durchgeführt werden.

13. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

14. Verwendung von Neurophysin II (SEQ ID No. 1) und ggfs. weitere Marker nach einem der Ansprüche 5 bis 10 zur in-vitro Diagnose und / oder Risikostratifizierung von Herzinsuffizienz.

15. Verwendung eines Kits zur in-vitro Diagnose und / oder Risikostratifizierung von Herzinsuffizienz, enthaltend Nachweisreagenzien zur Bestimmung des Markers Neurophysin II (SEQ ID No. 1) und ggfs. weitere Marker nach einem der Ansprüche 5 bis 9 und Hilfsmittel.

## Claims

1. A method for in vitro diagnosis and/or risk stratification of heart failure, **characterized in that**
a determination of the marker neurophysin II (SEQ ID no. 1) is carried out on a patient to be examined.

2. A method according to claim 1, **characterized in that** the heart failure comprises chronic heart failure, hypertensive heart disease with (congestive) heart failure, hypertensive heart and kidney disease with (congestive) heart failure, primary right heart failure, secondary right heart failure, left heart failure without complaints (NYHA stage I), left heart failure with complaints under strong load (NYHA stage II), left heart failure with complaints under light load (NYHA stage III), left heart failure with complaints at rest (NYHA stage IV).

3. A method for in-vitro diagnosis and/or risk stratification of heart failure according to any one of claims 1 through 2 for making clinical decisions, in particular about further treatment and therapy by means of drugs, especially in intensive medicine or emergency medicine and for hospitalization of the patient.

4. A method for in vitro diagnosis of heart failure according to any one of claims 1 through 3 for prognosis, for early differential diagnosis and detection, for assessment of the severity of heart failure, and for assessment of the clinical course accompanying its therapy.

5. A method according to any one of the preceding claims, **characterized in that** a determination is additionally carried out, on a patient to be examined, of at least one other marker selected from the group of inflammatory markers, cardiovascular markers, neurohormonal markers, or ischemic markers.

6. A method according to any one of the preceding claims, **characterized in that** the inflammatory marker is selected from at least one marker from the group C-reactive protein (CRP), cytokines, such as, for instance, TNF-alpha, interleukins, such as, for instance, IL-6, procalcitonin (1-116, 3-116), and adhesion molecules, such as VCAM or ICAM.

7. A method according to any one of the preceding claims, **characterized in that** the cardiovascular marker is selected from at least one marker from the group creatine kinase, myeloperoxidase, copeptin, myoglobin, natriuretic protein, especially ANP (or ANF), proANP, NT-proANP, BNP, proBNP, NT-proBNP or a partial sequence of any one of them, cardiac troponin, CRP and circulation-regulating prohormones, such as pro-gastrin-releasing peptide (proGRP), pro-endothelin 1, pro-leptin, pro-neuropeptide Y, pro-somatostatin, pro-neuropeptide YY, pro-opiomelanocortin, pro-adrenomedullin (proADM), copeptin, or a partial sequence of any one of them.

8. A method according to any one of the preceding claims, **characterized in that** the ischemic marker is selected from at least one marker from the group troponin I and T, and CK-MB.

9. A method according to any one of the preceding claims **characterized in that** the neurohormonal marker is at least one natriuretic peptide, in particular ANP (or ANF), proANP, NT-proANP, BNP, proBNP, NT-proBNP, or a partial sequence thereof.

10. A method according to any one of the preceding claims, **characterized in that** parallel or simultaneous determinations of the markers are carried out.

11. A method according to any one of the preceding claims, **characterized in that** the determinations are carried out on at least one patient sample.

12. A method according to any one of the preceding claims, **characterized in that** the determinations are carried out on an automated analyzer, in particular by means of a Kryptor.

13. A method according to any one of the preceding claims **characterized in that** the determinations are carried out by means of a rapid test, in particular in single parameter determinations or in multi-parameter determinations.

14. The use of neurophysin II (SEQ ID no. 1) and possibly other markers according to one of claims 5 through 10 for in vitro diagnosis and/or risk stratification of heart failure.

15. The use of a kit for in vitro diagnosis and/or risk stratification of heart failure, containing detection reagents for determination of the marker neurophysin II (SEQ ID no. 1) and possibly other markers according to any one of claims 5 through 9, and adjuvants.

## Revendications

1. Procédé de diagnostic in vitro et/ou de stratification de risques d'une insuffisance cardiaque, **caractérisé en ce**
**qu'**une détermination du marqueur neurophysine II (SEQ ID N° 1) d'un patient à examiner est exécutée.

2. Procédé selon la revendication 1, **caractérisé en ce que**
l'insuffisance cardiaque comprend une insuffisance cardiaque chronique, une maladie hypertensive avec une insuffisance cardiaque (congestive), une maladie hypertensive cardiaque et des reins avec une insuffisance cardiaque (congestive), une insuffisance cardiaque droite primaire, une insuffisance cardiaque droite secondaire, une insuffisance cardiaque gauche sans douleurs (NYHA - Stade I), une insuffisance cardiaque gauche avec des douleurs pour une sollicitation plus importante (NYHA-Stade II), une insuffisance cardiaque gauche avec des douleurs pour une sollicitation plus faible (NYHA-Stade III), une insuffisance cardiaque gauche avec des douleurs au repos (NYHA-Stade IV).

3. Procédé de diagnostic in vitro et/ou de stratification des risques d'une insuffisance cardiaque selon l'une des revendications 1 à 2 destiné à la prise de décisions cliniques, notamment de la poursuite du traitement et de la thérapie au moyen de médicaments, en particulier en médecine intensive ou en médecine d'urgence, et pour l'hospitalisation du patient.

4. Procédé de diagnostic in vitro d'une insuffisance cardiaque selon l'une des revendications 1 à 3, destiné au pronostic, à la reconnaissance précoce par diagnostic différentiel et à la reconnaissance, destiné à l'évaluation du degré de gravité et destiné à l'évaluation de l'évolution en accompagnement thérapeutique d'une insuffisance cardiaque.

5. Procédé selon l'une des revendications précédentes,
**caractérisé en ce**
**que**, complémentairement, une évaluation d'au moins un autre marqueur choisi dans le groupe des marqueurs inflammatoires, des marqueurs cardiovasculaires, des marqueurs neuro-hormonaux ou des marqueurs ischémiques est exécutée sur un patient à examiner.

6. Procédé selon l'une des revendications précédentes, **caractérisé en ce que** le marqueur inflammatoire est choisi à partir au minimum d'un marqueur du groupe de la protéine C réactive (CRP), des cytokines, comme, par exemple, le TNF alpha, des interleukines, comme, par exemple, l'IL-6, de la procalcitine (1-116, 3-116) et de molécule d'adhésion comme la VCAM ou l'ICAM.

7. Procédé selon l'une des revendications précédentes, **caractérisé en ce que** le marqueur cardiovasculaire est choisi à partir d'au moins un marqueur du groupe de la créatine kinase, de la myéloperoxydase, de la copeptine, de la myoglobine, de la protéine natriurétique, notamment de l'ANP (respectivement, l'ANF), de la proANP, de la NT-proANP, de la BNP, de la proBNP, de la NT-proBNP ou chaque fois d'une séquence partielle de celles-ci, de la troponine cardiaque, de la CRP ainsi que de (pro)hormones régulatrices du système circulatoire, comme le peptide libérant la pro-gastrine (proGRP), la pro-endothéline-1, la pro-leptine, le pro-neuropeptide-Y, la pro-somatostatine, le pro-neuropeptide-YY, la pro-opiomélanocortine, la pro-adrénomédulline (proADM), la copeptine ou chaque fois une séquence partielle de ceux-ci.

8. Procédé selon l'une des revendications précédentes, **caractérisé en ce que** le marqueur ischémique est choisi à partir d'au moins un marqueur du groupe des troponines I et T, de la CK-MB.

9. Procédé selon l'une des revendications précédentes, **caractérisé en ce que** le marqueur neuro-hormonal est au moins une protéine natriurétique, notamment l'ANP (respectivement, l'ANF), la proANP, la NT-proANP, la BNP, la proBNP, la NT-proBNP ou une séquence partielle de celles-ci.

10. Procédé selon l'une des revendications précédentes,
**caractérisé en ce**
**que** des évaluations parallèles ou simultanées des marqueurs sont effectuées.

11. Procédé selon l'une des revendications précédentes,
**caractérisé en ce**
**que** les évaluations sont effectuées sur au moins un échantillon du patient.

12. Procédé selon l'une des revendications précédentes,
**caractérisé en ce**
**que** les évaluations sont exécutées sur un automate d'analyses, notamment au moyen d'un analyseur Kryptor.

13. Procédé selon l'une des revendications précédentes,
**caractérisé en ce que** les évaluations sont effectuées au moyen d'un test rapide, notamment dans des évaluations d'un paramètre individuel ou de plusieurs paramètres.

14. Utilisation de neurophysine II (SEQ ID N° 1) et éventuellement, d'autres marqueurs selon l'une des revendications 5 à 10 pour le diagnostic in vitro et/ou de la stratification des risques d'une insuffisance cardiaque.

15. Utilisation d'un kit pour le diagnostic in vitro et/ou la stratification des risques d'une insuffisance cardiaque, contenant des réactifs indicateurs pour l'évaluation du marqueur neurophysine II (SEQ ID N° 1) et éventuellement d'autres marqueurs selon l'une des revendications 5 à 9, et des produits auxiliaires.
